# EUROPEAN PATENT SPECIFICATION

(11) **EP 4 355 392 B1**
(45) Date of publication and mention of the grant of the patent: **29.07.2026**
(21) Application number: 22731784.9
(22) Date of filing: 13.06.2022
(51) Int. Cl.: A61M 5/19, A61M 5/28, A61M 5/31, A61B 17/00

(54) **INJECTION DEVICE FOR INJECTION OF BIOLOGICAL MATERIALS**
INJEKTIONSVORRICHTUNG FÜR DIE INJEKTION VON BIOLOGISCHEM MATERIAL
DISPOSITIF D'INJECTION POUR L'INJECTION DE MATIERES BIOLOGIQUES

(30) Priority: 14.06.2021 GB 202108461
(43) Date of publication of application: 24.04.2024
(73) Proprietor: Imperial College Innovations Limited, London SW7 2AZ (GB)
(72) Inventor: MOORE, James, London Exhibition Road South Kensington London SW7 2AZ (GB)
(74) Representative: Pitchford, James Edward
(86) International application number: PCT/GB2022/051479
(87) International publication number: WO 2022/263797

(56) References cited:
- WO-A2-2007/006030
- US-A1- 2007 191 781
- US-A1- 2008 105 318

## Description

### Field of the Invention

This invention relates to a multi-compartment injection device, particularly (but not necessarily) in the form of a syringe, for the injection of biological materials such as biological cells, for example.

### Background to the Invention

Injection devices such as syringes may be used to inject or otherwise deliver a carrier fluid containing cells or other biological material(s) for medical, biological, research or manufacturing purposes. To this end, the injection device may commonly (but not always) be fitted with a needle.

The passage of the cell suspension through the small bore of the needle has been demonstrated to cause a large percentage of the cells to die, presumably due to the mechanical environment they encounter [1, 2]. This is a particular problem for cell therapy procedures, in which small-bore needles are required to reach the target therapeutic area. The needle normally possesses the smallest cross-sectional area of any part of the injection device, and therefore features the highest fluid flow velocities (by conservation of mass). The low cell viability rates in some previous experiments have been attributed to "shear effects" experienced by the cells. In the case of flow down a long, small diameter tube, the major component of the shear stress varies linearly from 0 at the centre of the tube to a maximum value at the wall of the tube. Therefore, it is presumably the cells located towards the outer portions of the cross-section that are most likely to be killed.

A similar issue is also encountered in the practice of manufacturing 3D tissues containing cells using bioplotting, which usually involves the passage of cell suspensions down a needle that is manoeuvred in 3D using high-resolution robotics.

Accordingly, with the above examples, the shear effects experienced by the carrier fluid typically result in only a small percentage of the cells that were prepared for injection/delivery actually surviving the injection/delivery process. This leads to wasted cells and reduced effectiveness of therapies such as stem cell therapies. The "shear effects" in question, particularly in respect of small-bore needle injections, have generally been assumed to be shear stress [3, 4, 5].

There is therefore a desire to reduce the damage that is done to biological materials such as cells during such an injection/delivery process, and thereby improve the survivability of the biological materials.

Background art is provided in WO 2013/070692 A1, which discloses a multi-compartment syringe for delivering a high viscosity carrier fluid containing proteins from a first syringe compartment, and a low viscosity lubricating fluid from a separate second syringe compartment. This is with a view to reducing the injection force needed to deliver the carrier fluid. However, WO 2013/070692 A1 does not address the issue of shear effects or the damage caused by shear effects on biological cells in particular.

Further background art is provided in US 2007/191781 A1, US 2008/105318 A1 and WO 2007/006030 A2.

US 2007/191781 A1 discloses an apparatus for percutaneous delivery of a sealant, the apparatus comprising: at least two fluid reservoirs, an introducer needle having a distal tip that is in fluid communication with at least one reservoir, and a fluid delivery tube that is in fluid communication with a second reservoir, wherein the fluid delivery tube has a tip and wherein the fluid delivery tube is configured so that the tip of the fluid delivery tube extends to but not beyond the distal tip of the introducer needle during use.

US 2008/105318 A1 discloses a fluid turbulence minimizing device comprising: a connection portion defining a connection cavity shaped to receive a tertiary fluid-supply conduit including a primary fluid-supply lumen and two secondary fluid-supply lumens; and an intermixing portion having a longitudinal flow axis and defining an intermixing cavity that has an input orifice fluidically communicating with the connection cavity and a given area, an exit orifice having an area less than the given area, an inner surface having an upstream side adjacent the connection portion, a downstream side at a distance from the connection portion, and a cross-sectional area decreasing from the input orifice to the exit orifice to convey fluid supplied from the conduit to the connection cavity through the intermixing cavity and out the exit orifice, and guide fins inwardly projecting from the inner surface of the intermixing portion toward the longitudinal flow axis and having a longitudinal extent aligned substantially parallel with the longitudinal flow axis

WO 2007/006030 A2 discloses a dual chamber syringe comprising: an inner cylindrical body having an open end, a closed end with an inner discharge outlet formed therein, and an inner plunger sealingly slideable within the inner body; and an outer cylindrical body enclosing the inner cylindrical body and having an open end, a closed end with an outer discharge outlet formed therein, and an outer plunger sealingly slidable between the outer body and the inner body, the inner discharge outlet extending into the outer discharge outlet such that the inner body is in fluid communication through the outer discharge outlet with the exterior of the inner and outer body.

### Summary of the Invention

The present invention is defined in the appended claims. Details of certain embodiments are set out in the dependent claims.

More particularly, according to a first aspect of the present invention there is provided an injection device as defined in Claim 1 of the appended claims.

Thus there is provided an injection device comprising: a first barrel for containing a first fluid, and having a first plunger; a second barrel for containing a second fluid, and having a second plunger, the second barrel being arranged to one side of the first barrel; a flow converging chamber in fluid communication with the first and second barrels, for receiving, in use, a flow of the first fluid from the first barrel via a first inlet to the flow converging chamber when the first plunger is advanced within the first barrel, and a flow of the second fluid from the second barrel via a second inlet to the flow converging chamber when the second plunger is advanced within the second barrel, simultaneously with the advancement of the first plunger, the first inlet being arranged to introduce the flow of the first fluid in a laminar concentric manner within the flow of the second fluid, at a meeting point within the flow converging chamber, to produce a laminar concentric flow of the first fluid within the second fluid; and an outlet of the flow converging chamber, for outwardly delivering the laminar concentric flow of the first fluid within the second fluid; wherein the first inlet is in the form of a barrel, of which a downstream part extends within the flow converging chamber, towards the outlet, and wherein the second inlet is upstream of the meeting point and alongside the downstream part of the first inlet and arranged such that, in use, at the meeting point, the first fluid and the second fluid are both already flowing in the direction of the outlet; and wherein the flow converging chamber tapers towards the outlet, the taper of the flow converging chamber beginning at, or downstream of, the meeting point.

Advantageously, the first fluid may contain cells or other biological materials. The injection device may be used to deliver the first fluid, concentrically surrounded by the second fluid, in a manner that minimises the shear rate experienced by the first fluid. In this regard, the present inventor has found that it is excessive shear rate, and not excessive shear stress, that causes damage to cells. By minimising the shear rate to which the first fluid is subjected, the present injection device reduces the damage that is done to the cells (or other biological materials) during the injection/delivery process, and thereby improves the survivability of the cells (or other biological materials).

Further, by virtue of the second barrel being arranged to one side of the first barrel, this enables the second barrel to be made significantly smaller than the first barrel (in terms of diameter and thus volume), whilst still enabling the device to be made at reasonable cost, e.g. using common plastics materials and straightforward manufacturing processes. Moreover, by making the second barrel smaller than the first barrel, this enables the device to deliver only a required minimum amount of the second fluid, no more than is necessary, which in turn reduces the likelihood of detrimental effects that may otherwise arise as a consequence of using an excessive quantity of the second fluid. These effects include causing mechanical trauma to the surrounding tissues during cell injection procedures or reduced structural integrity in bioplotting constructs.

In accordance with the invention, the first inlet extends within the flow converging chamber, towards the outlet, such that, in use, at the meeting point, the first fluid and the second fluid are both already flowing in the direction of the outlet, ideally parallel to one another. It is further beneficial to have the two fluids meet with similar velocities, and such that they make a minimal transition from their individual passageways to the interfacial shear stresses encountered after they meet. In situations involving two fluids flowing parallel to one another and in contact, the velocity and shear stress are continuous across the fluid interface. Therefore, easing the transition to this state at the meeting point reduces the likelihood of mixing of the first and second fluids as the concentric flow of the first fluid within the second fluid is formed.

In accordance with the invention, the flow converging chamber tapers towards the outlet, thereby further reducing the risk of fluid mixing as the concentric flow of the first fluid within the second fluid passes towards the outlet. The taper of the flow converging chamber begins at, or downstream of, the meeting point.

In certain embodiments the taper of the flow converging chamber may decrease linearly. However, in other embodiments the radius r of the tapering section may be inversely proportional to a function of the distance z towards the outlet.

In particular, the radius r of the tapering section may be inversely proportional to the square root of z (i.e. such that the radius r is substantially proportional to z^{-½}). Such a geometry minimises the peak viscous normal stress and strain rate encountered by the first fluid at any location along the flow pathway. This may have additional benefits of minimising the risk of fluid mixing as well as minimising the likelihood of cell damage.

Preferably the first inlet and the outlet are substantially axially aligned with the first barrel, thereby enabling the first fluid to pass through the flow converging chamber without deviating from a linear path.

Moreover, preferably, when viewed in longitudinal cross section: the second inlet meets the flow converging chamber on a first side of the flow converging chamber; the flow converging chamber has a second side opposite the first side; the flow converging chamber has a first flow path width between a first side wall of the downstream part of the first inlet and an outer wall of the flow converging chamber on the first side; the flow converging chamber has a second flow path width between a second side wall of the downstream part of the first inlet and an outer wall of the flow converging chamber on the second side; and the second flow path width is greater than the first flow path width. Preferably the second flow path width increases in a direction upstream from the meeting point, away from the outlet. Preferably the second flow path width is substantially equal to the first flow path width at the meeting point. Advantageously, such a configuration (in which an upper part of the outer wall of the flow converging chamber is effectively skewed outwardly on the second side, opposite the point where the second inlet meets the flow converging chamber on the first side) enables an axisymmetric pressure profile to be achieved in the second fluid when it reaches the meeting point.

The first and second barrels may be parallel to one another, or at an angle to one another but still coplanar, or oriented at a non-coplanar angle with respect to one another. These configurations could be applied to optimise the ergonomics of the user interface or incorporation in a particular bioplotter or other device.

Optionally at least some of the internal surfaces of the injection device may be superhydrophobic, to minimise the shear rate encountered by the fluids flowing through the device.

Optionally the injection device may further comprise a needle in fluid communication with the outlet.

In certain embodiments the first and second plungers may be connected to each other, thereby facilitating manual operation and enabling the plungers to be advanced simultaneously with equal velocity. However, for other embodiments the first and second plungers need not be connected to each other, and indeed may be arranged to be actuated some other way, for example by means of motors (e.g. in order to drive the plungers at different velocities from one another).

As mentioned above, the first fluid may contain biological materials such as biological cells. Preferably the first fluid has a higher viscosity than that of the second fluid. Preferably the first fluid exhibits a yield stress. For example, the first fluid may comprise a hydrogel. The second fluid may comprise an aqueous fluid such as phosphate-buffered saline (PBS). It is also possible to include additives to the fluids to reduce their miscibility, but it would be undesirable to include additives that reduce cell viability via biochemical actions.

For ease of manufacturing and to reduce costs, one or more of the first barrel, the first plunger, the second barrel, the second plunger and the flow converging chamber may be formed of a plastics material, for example by injection moulding.

The first inlet (by means of which the flow of the first fluid is introduced in a concentric manner within the flow of the second fluid) may be formed of a metal such as stainless steel, to enable its walls to be made as thin as possible, thereby reducing off-axis fluid velocities and mixing as the first and second fluids come together at the meeting point.

According to a second aspect of the invention there is provided a method of delivering a concentric flow of a first fluid within a second fluid, using an injection device according to the first aspect of the invention.

The first fluid may contain biological materials such as biological cells.

Preferably the first fluid has a higher viscosity than that of the second fluid.

Preferably the first fluid exhibits a yield stress.

For example, the first fluid may comprise a hydrogel. The second fluid may comprise an aqueous fluid such as phosphate-buffered saline.

Preferably, at the meeting point, the second fluid has an axisymmetric pressure profile.

Preferably, to avoid damaging the contents of the first fluid, the first fluid is subjected to a shear rate well below the critical shear rate of the cells (i.e. the shear rate at which damage is caused to the cells) as the first fluid passes through the flow converging chamber and the outlet. Ideally the first fluid is subjected to a shear rate of substantially zero as it passes through the flow converging chamber and the outlet.

### Brief Description of the Drawings

Embodiments of the invention will now be described, by way of example only, and with reference to the drawings in which:
Figure 1 illustrates the principle of laminar flow, which involves successive layers of fluid sliding past one another;
Figure 2 is a schematic plot of shear stress against shear rate for three illustrative fluids exhibiting different viscosities and viscous behaviours (namely Newtonian and Power-Law fluids);
Figure 3 illustrates the principles of fluid shear stress when a fluid flows through a cylinder;
Figure 4 is a schematic plot of shear stress against radial position for fluid flowing in a cylinder;
Figure 5 is a schematic plot of shear rate against radial position for Newtonian and Power-Law fluids flowing in a cylinder;
Figure 6 illustrates experimental results showing variation in biological cell viability rate following syringe/needle injections as functions of (a) wall shear rate, and (b) wall shear stress;
Figure 7 illustrates examples of two concentric fluids, for example a hydrogel (containing biological cells) surrounded by phosphate-buffered saline (PBS) flowing in a cylinder;
Figure 8 is a schematic plot of shear rate against radial position across the concentric fluids of Figure 7;
Figure 9 illustrates an injection device in the form of a syringe, according to an embodiment of the present invention, having first and second barrels in a parallel side-by-side configuration, for containing first and second fluids respectively, and a flow converging chamber arranged to produce a concentric flow of the first fluid (via a first inlet) within the second fluid;
Figure 10 illustrates an exploded view of the syringe of Figure 9;
Figure 11 illustrates a longitudinal cross-sectional view of the flow converging chamber of Figure 9;
Figure 12 illustrates a longitudinal cross-sectional view of the flow paths of Figure 11, annotated to indicate exemplary dimensions;
Figure 13 illustrates another longitudinal cross-sectional view of the flow converging chamber of Figure 9, axially rotated through 90° relative to Figure 11, and annotated to indicate exemplary dimensions;
Figure 14 illustrates a longitudinal cross-sectional view of the first and second fluids flowing through the flow converging chamber;
Figure 15 is a close-up view of the flowing first and second fluids of Figure 14, in the vicinity of the meeting point of the first and second fluids, together with transverse cross-sectional views of the first and second fluids (a) before the meeting point, and (b) at the meeting point;
Figure 16 is a close-up view of the flowing first and second fluids of Figure 14, in a tapering section of the flow converging chamber; and
Figure 17 is a sketch of the flowing first and second fluids in an alternative configuration of the tapering section of the flow converging chamber.

In the figures, like elements are indicated by like reference numerals throughout.

### Detailed Description of Preferred Embodiments

The present embodiments represent the best ways known to the Applicant of putting the invention into practice. However, they are not the only ways in which this can be achieved.

### Technical background

Low cell viabilities (<50%) following injection through small-bore needles has traditionally been attributed to excessive levels of shear stress [3, 4, 5]. However, as part of the present work, we have determined that it is actually excessive shear rate (and not excessive shear stress) during the injection process that causes death of cells and likewise damage to other biological materials.

The objective of subjecting the cells (or other biological materials) to a sufficiently low shear rate during the injection process has served as a design criterion in the present work, with a view to achieving cell viability rates of 85% or greater while maintaining injection velocities low enough to encourage widespread clinical and bioplotting use.

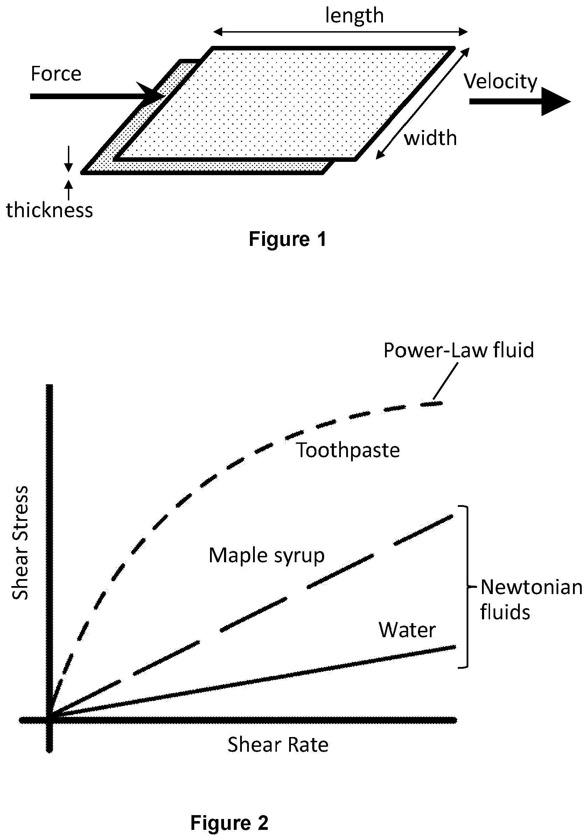

As background, to explain the difference between shear rate and shear stress, Figure 1 illustrates the principle of laminar flow, which involves successive layers of fluid sliding past one another. On a very simplistic level, this may be considered analogous to one sheet of plywood sliding over another.

With reference to Figure 1, Shear Rate is the kinematic quantity related to the velocity of one layer of fluid relative to the next (i.e. difference in velocity / thickness).

On the other hand, Shear Stress is given by the force required to make one layer slide relative to another, divided by the surface area (i.e. force / (length x width)). The ratio of shear stress to shear rate is the viscosity of the fluid.

Turning now to Figure 2, this shows a schematic plot of shear stress against shear rate for three illustrative fluids, namely toothpaste, maple syrup and water.

The simplest fluids are those with a constant viscosity, represented by a constant slope straight line in the shear stress vs shear rate plot. These are called Newtonian fluids. Maple syrup and water are examples of Newtonian fluids. Maple syrup has a higher viscosity than water, so it takes more shear stress to achieve the same shear rate.

Other fluids, including toothpaste, have a more complex, nonlinear viscous behaviour. Toothpaste, and other fluids having similar viscous behaviour, such as many hydrogels, are examples of Power-Law fluids.

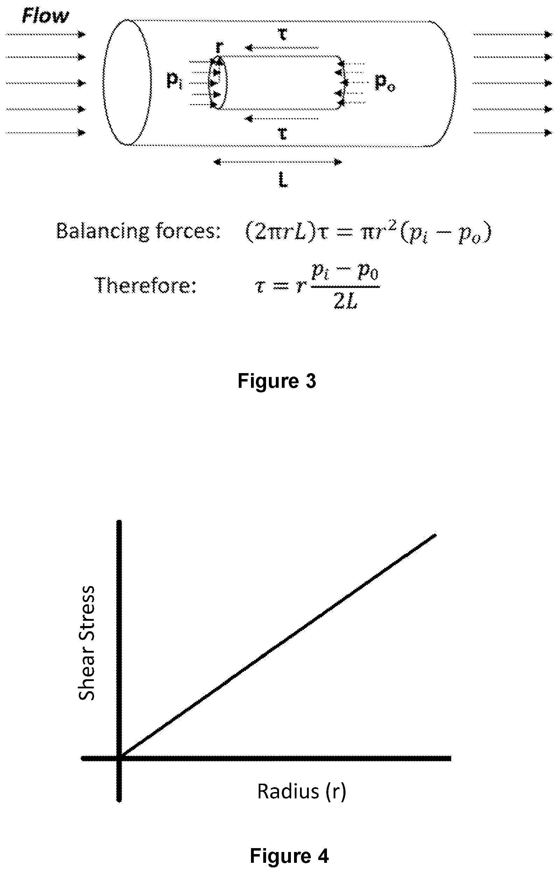

Figure 3 illustrates the principles of fluid shear stress in a cylinder under conditions equivalent to Poiseuille flow (fully developed axisymmetric steady laminar flow). The symbols used are as follows:
*τ* = the radial-axial component of the shear stress tensor (the other components are zero under Poiseuille flow conditions)
*p* = pressure
*r* = radius
*L* = axial length

A force balance on a cylindrical volume of injected liquid demonstrates the linear dependence of shear stress ***τ*** on radial position r, regardless of the properties of the fluid. This is illustrated in the plot shown in Figure 4. This equation applies anywhere in the fluid, including at the tube wall.

Previous research (e.g. [2]) demonstrated that using different suspension fluids had an effect on viability rates. Therefore, cell deaths are not likely caused by excessive shear stress. If shear stress were the cause of cell deaths, there should have been no effect of using a different fluid. Furthermore, in those studies it was often noted that using the higher viscosity fluid (e.g., hydrogels) resulted in a higher viability rate. A higher viscosity fluid would have required a higher pressure gradient to move the same volume flow rate of fluid through the needle. According to the equation in Figure 3, a higher axial pressure gradient (pᵢ-pₒ)/L implies higher shear stress, which is counter to the conclusion that excessive shear stress causes cell deaths.

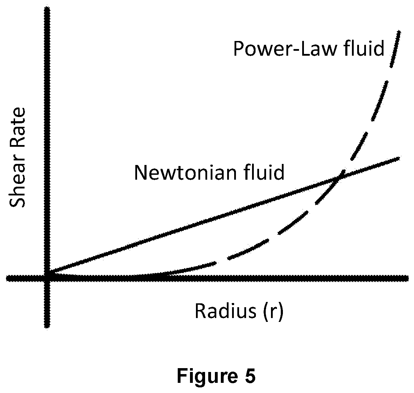

Figure 5 is a schematic plot of shear rate against radius (radial position) for Newtonian and Power-Law fluids under conditions equivalent to Poiseuille flow.

With Newtonian fluids, shear rate is proportional to shear stress, so that also depends linearly on radial position (as shown in Figure 5).

On the other hand, shear thinning fluids becomes less viscous as shear rate increases. Many hydrogels behave in this manner, for instance, and the shear rate - shear stress relationship can be described with a power law fluid model. For such a fluid, shear rate is lower in the middle of the tube and much higher (in a non-linear manner) near the wall (as shown in Figure 5). The "plug flow" of toothpaste emerging from a tube is a common example of this.

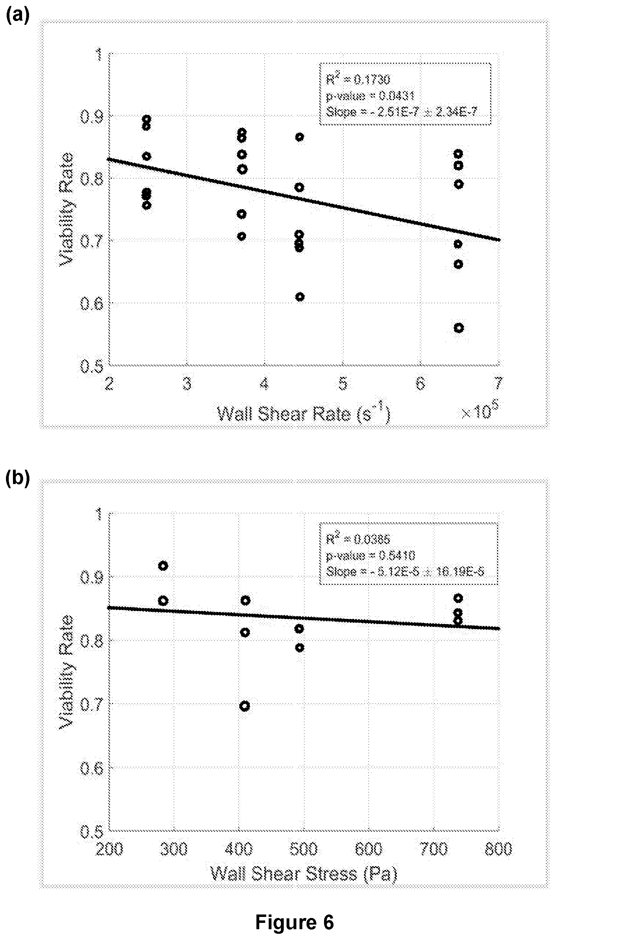

Figure 6 illustrates experimental results showing variation in biological cell viability rate with (a) wall shear rate, and (b) wall shear stress. From Figure 6(a), a statistically significant negative slope was found between viability rate and shear rate. On the other hand, from Figure 6(b), no statistically significant slope was demonstrated between viability rate and shear stress. This supports our hypothesis that it is excessive shear rate, and not shear stress, during the injection process that causes death of cells and potentially damage to other biological materials.

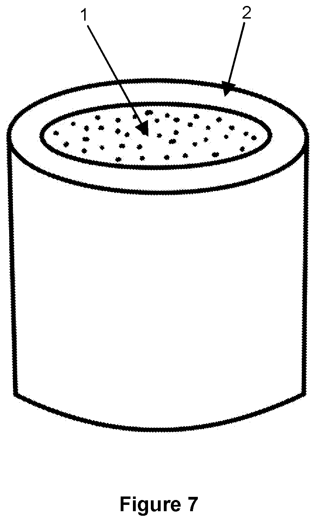

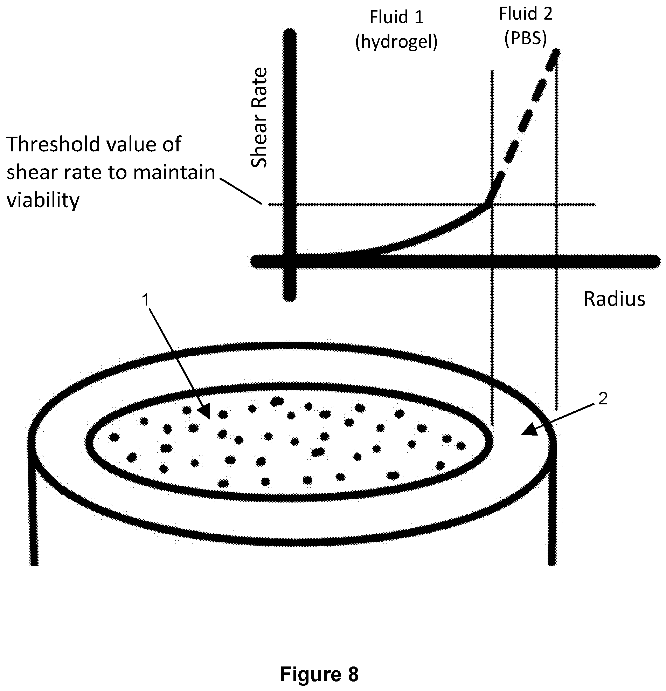

Having established what kills cells, we have been able to create an injection device to minimise the shear rate to which biological materials such as cells are subjected. With reference to Figures 7 and 8, the objective is to have the biological materials (e.g. cells) contained in an inner (first) fluid 1 that exhibits a yield stress, for example a hydrogel, that behaves as a power law fluid. The objective of the design is to maintain a shear rate in the inner fluid 1 that is below the critical shear rate for the cells or biological material contained in the fluid throughout the injection process. The inner fluid 1 is concentrically surrounded by an outer (second) water-based fluid 2, such as phosphate-buffered saline (PBS), which contains no cells/biological materials. The outer fluid 2 may be considered to be a lubricating fluid or sheath fluid. Its function is merely to bear the higher shear rates adjacent the internal walls of the injection device/needle, and can be very thin. Using properties of existing hydrogels and needle bores, we estimate that the thickness of the layer of the outer fluid 2 only needs to be approximately <5% of the radius of the needle in order to maintain the shear rate below the critical shear rate identified in the experiments that also yielded Figure 6.

Thus, in summary, our objective was to create an injection device that provides a concentric outer layer of fluid 2 that minimises the shear rate in the inner fluid 1. The outer fluid 2 (PBS) takes up most of the shear rate, while the inner fluid 1 (cell-containing hydrogel) is subjected to only low shear rates. In such a manner, the inner fluid 1 (cell-containing hydrogel) can be maintained at shear rates that do not exceed a threshold value at which cell viability is significantly affected, thereby enabling a greater proportion of the cells to survive the injection process than would otherwise be the case. Ideally the inner fluid 1 would have a shear rate of substantially zero, such that the inner fluid 1 moves as a solid core, and none of the cells in the inner fluid 1 would be lost to shear rate-induced damage.

### Illustrative embodiments of the present injection device

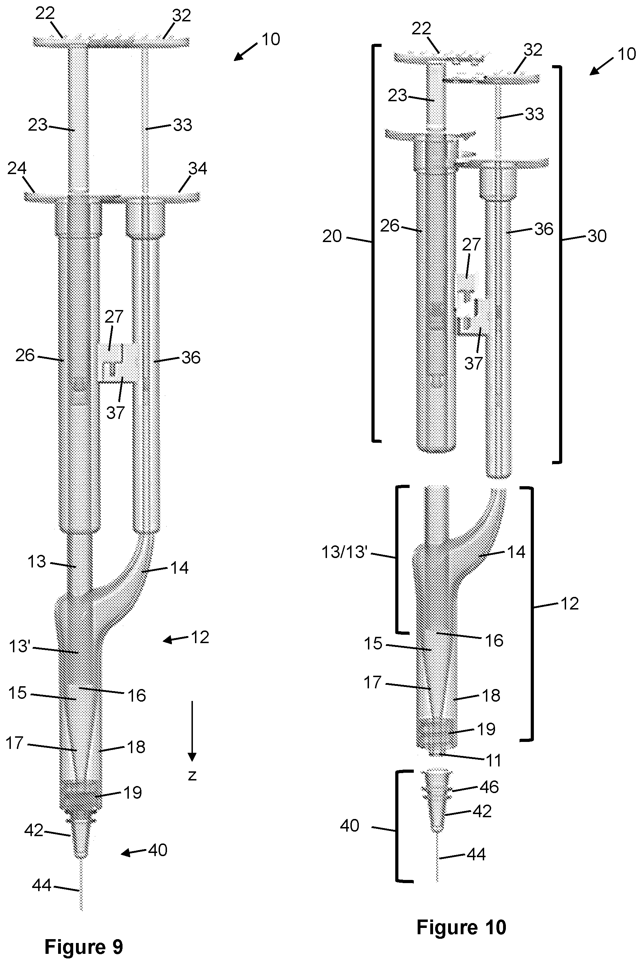

Consistent with the above objectives, and with reference initially to Figures 9 and 10, the present disclosure provides an injection device 10, for injection of biological materials (e.g. cells) within a first fluid 1, surrounded by a second fluid 2. In the illustrated embodiment the injection device is in the form of a multi-compartment syringe 10, but the principles (particularly with respect to the arrangement of the compartments/barrels and the fluid paths) may be readily applied to forms of injection devices other than syringes.

As illustrated, the syringe 10 comprises a first barrel 26 for containing the first fluid 1 (e.g. hydrogel), and having a first plunger 23. In use, the biological materials (e.g. cells) are provided in the first fluid 1, within the first barrel 26. The syringe 10 further comprises a second barrel 36 for containing the second fluid 2 (e.g. PBS), and having a second plunger 33. The second barrel 36 is arranged to one side of the first barrel 26, i.e. in a side-by-side manner, and accordingly the second plunger 33 is also arranged to one side of the first plunger 23. The first and second barrels 26, 36 are both cylindrical in shape, i.e. are of circular cross-section in the example shown, but other cross-sectional shapes can be used. Moreover, it will be appreciated that, in the illustrated embodiment, the first and second barrels 26, 36 are parallel to one another. However, in alternative embodiments, the first and second barrels 26, 36 may be at an angle to one another but still coplanar, or oriented at a non-coplanar angle with respect to one another. These configurations could be applied to optimise the ergonomics of the user interface or incorporation in a particular bioplotter or other device.

In the illustrated embodiment the first and second plungers 23, 33 are respectively provided with first and second plunger flanges 22, 32. Likewise, the first and barrels 26, 36 are respectively provided with first and second barrel flanges 24, 34. The first and second barrels 26, 36 are connected to each other, side by side, by means of the barrel flanges 24, 34 interlocking with one another, and also by means of interlocking parts 27, 37 provided part way down the barrels (with part 27 being attached to the first barrel 26, part 37 being attached to the second barrel 36, and parts 27 and 37 subsequently being engaged with one another). The first and second plunger flanges 22, 32 are also interlocked with one another, to enable simultaneous advancement of the first and second plungers 23, 33 by finger pressure on the plunger flanges 22, 32. Conveniently, the connected plunger flanges 22, 32 may permit single handed operation of the syringe 10. More particularly, by moving the connected first and second plunger flanges 22, 32 towards the first and second barrel flanges 24, 34, the first and second plungers 23, 33 move at the same velocity, and the first fluid 1 is ejected from the first barrel 26 simultaneously with the ejection of the second fluid 2 from the second barrel 36.

In alternative embodiments, however, the first and second plungers 23, 33 need not be provided with finger-operable plunger flanges 22, 32, and may instead be arranged to be actuated some other way, for example by means of motors (e.g. stepper motors) which act on the first and second plungers 23, 33. Such motors may be used to drive the first and second plungers 23, 33 at different velocities from one another, if desired.

The first and second plungers 23, 33 are each terminated by respective plunger tips, that form a seal within the first and second barrels 26, 36 respectively, and, when advanced, apply pressure on the fluids within the first and second barrels 26, 36 to cause the fluids therein to be ejected.

The syringe 10 comprises a flow converging chamber 15 in fluid communication with the second barrel 36, for receiving, in use, a flow of the second fluid 2 from the second barrel 36 when the second plunger 33 is advanced within the second barrel 36.

The syringe 10 further comprises a first inlet 13 to the flow converging chamber 15, in communication with the first barrel 26, for receiving, in use, a flow of the first fluid 1 from the first barrel 26 when the first plunger 23 is advanced within the first barrel 26, simultaneously with the advancement of the second plunger 33. The first inlet 13 is arranged to introduce the flow of the first fluid 1 in a concentric manner within the flow of the second fluid 2, at a meeting point 16 within the converging chamber 15, to produce a concentric flow of the first fluid 1 within the second fluid 2. Accordingly, the flows of the first and second fluids 1, 2 are concentric at the point where they come together (the meeting point 16), and remain concentric as they pass through the rest of the syringe 10.

The flow converging chamber 15 also has an outlet 11 for outwardly delivering the concentric flow of the first fluid 1 within the second fluid 2, for example to an attached needle 40. In the illustrated embodiment the flow converging chamber 15 tapers towards the outlet 11, thus providing a tapering section 17. The tapering section 17 begins at, or downstream of, the meeting point 16.

In the illustrated embodiment the tapering section 17 has a radius r that varies with distance z along the tapering section towards the outlet 11, in a manner such that the radius r decreases linearly with increasing z. However, in alternative embodiments, the radius r of the tapering section may be inversely proportional to a function of the distance z. For instance, the radius r of the tapering section may be inversely proportional to the square root of z (i.e. such that the radius r is substantially proportional to z^{-½}), as is discussed in greater detail below.

Radially outward of the tapering section 17, an outer wall 18 of the device is straight sided (i.e. is of constant external diameter), and may for example be formed of solid plastic. This provides additional strength to the bottom region of the device, and may also provide a point of attachment for a needle 40.

In the illustrated embodiment of the syringe 10, an attachable/detachable needle 40 is attached to the outlet 11. The needle 40 comprises a needle shaft 44 and a needle hub 42. The needle hub 42 is configured to engage with the syringe 10, at the outlet 11, in a "Luer-lock" manner, by means of flanges 46 on the needle hub 42 engaging with threads 19 provided within the bottom of the outer wall 18, around the outlet 11. The needle 40 may for example be for the purpose of injection, e.g. for therapeutic purposes, or for bioprinting. Alternatively, instead of using a standard Luer-lock needle, the syringe 10 could include its own non-detachable needle. This would provide greater control of the pathways through which the fluids flow.

Notably, in the illustrated embodiment, the second barrel 36 is of smaller diameter (i.e. smaller volume) than the first barrel 26. Advantageously this enables the second fluid 2 to have a smaller volume flow rate compared to the first fluid 1, in the concentric flow of the second fluid 2 around the first fluid 1. In turn, this enables only a required minimum amount of the second fluid 2 to be used, no more than is necessary. Thus, only a thin outer layer of the second fluid 2 is formed around the first fluid 1. Minimising the volume of the second fluid 2 is desirable from a therapeutic point of view since additional fluid may induce extra tissue damage on injection, or may be detrimental to a bioprinting process.

Thus, the second barrel 36 is of smaller diameter (i.e. smaller volume) than the first barrel 26, to accommodate the smaller volume of second fluid 2 that is required. When using hydrogel as the first fluid 1 and an aqueous fluid such as PBS as the second fluid 2, we have estimated that we only need a very thin layer of the aqueous fluid, of the order of <5% of the radius of the needle.

The ability to make the second barrel 36 sufficiently small in comparison to the first barrel 26 is greatly facilitated by the second barrel 36 being arranged to one side of the first barrel 26, i.e. as two separate barrels side-by-side, rather than arranging them concentrically with the second barrel surrounding the first barrel (as is done in WO 2013/070692 A1, for example). Indeed, a side-by-side arrangement of barrels is relatively simple to make, at reasonable cost, from common plastics materials using straightforward manufacturing processes (e.g. injection moulding). Such a side-by-side arrangement of barrels is also reliable in use. On the other hand, a concentric arrangement of barrels is harder and more expensive to manufacture, particularly if the outer barrel is to be small in volume compared to the inner barrel. For instance, if one were to use a 1cm radius version of the concentric barrel design of WO 2013/070692 A1 with a 30G needle and aim to have 5% of the radius occupied by fluid 2 in the needle, then the outer layer of the syringe would need to be ≤0.03 µm thick. In addition to the challenges of mass-producing a structure of that size within a 1cm radius syringe, it would necessitate the use of materials such as engineered steel instead of plastics to form the barrels and plungers, adding to the manufacturing cost.

In other words, compared to a concentric barrel design such as that of WO 2013/070692 A1, the syringe 10 of the present work benefits from having the first and second barrels 26, 36 side-by-side (simplifying manufacture and increasing reliability, whilst enabling the second barrel 36 to be small in volume in comparison to the first barrel 26), whilst nevertheless delivering the flows of the first and second fluids 1, 2 in a concentric manner at the point where they come together (the meeting point 16) and enabling them to remain concentric as they pass through the rest of the syringe 10.

In the illustrated embodiment of the syringe 10, the first inlet 13 extends within the flow converging chamber 15, towards the outlet 11, such that, in use, at the meeting point 16, the first fluid 1 and the second fluid 2 are both already flowing in the direction of the outlet 11, ideally parallel to one another, and ideally such that the two fluids meet with similar velocities. This further reduces the likelihood of mixing of the first and second fluids as the concentric flow of the first fluid 1 within the second fluid 2 is formed. In passing, we note that the objective of the present work to reduce the likelihood of the first and second fluids mixing and to keep them in a concentric arrangement (from the meeting point 16 onwards) is fundamentally different from other two-barrel syringes, such as those used for epoxy cements, which are specifically designed to produce a uniform mixture of the two fluids.

To this end, according to the invention, the first inlet 13 is in the form of a barrel, of which a downstream part 13' extends within the flow converging chamber 15. The overall inlet barrel 13/13' may be integral with, or a continuation of, or attached to, the first barrel 26. Preferably the walls of the inlet barrel 13/13' are as thin as possible (e.g. 0.5 mm thick, or less), to reduce off-axis fluid velocities and mixing as the first and second fluids come together at the meeting point 16 within the flow converging chamber 15. The flow converging chamber passes 15 upwardly surrounds the downstream part 13' of the first inlet (as well as being beneath it), forming a narrow annular flow path around part 13'. Further features of the shape and configuration of the flow converging chamber 15 are described in greater detail below.

In the illustrated embodiment, the first inlet 13 (i.e. inlet barrel 13/13') and the outlet 11 are substantially axially aligned with the first barrel 26, to enable the first fluid 1 to pass through the flow converging chamber 15 without deviating from a linear path. Since the second barrel 36 is to one side of the first barrel 26, to convey the ejected second fluid 2 from the second barrel 36 to the flow converging chamber 15, a second inlet 14 to the flow converging chamber 15 is provided, on a first side of the flow converging chamber 15, at the top of the flow converging chamber 15 upstream of the meeting point 16, and to one side of part 13'. The second inlet 14 is in fluid communication with the second barrel 36 and, in use, conveys the second fluid 2 across from the second barrel 36 to the flow converging chamber 15.

In use, the first fluid 1 (e.g. hydrogel) flowing through the first barrel 26 and the first inlet (barrel 13/13') are initially subjected to shear rates well below the threshold for cell damage due to the diameters of the first barrel 26 and the first inlet (barrel 13/13') being sufficiently large.

The flow converging chamber 15 then allows the first fluid 1 and the second fluid 2 to meet concentrically, whilst minimising the likelihood of the two fluids mixing.

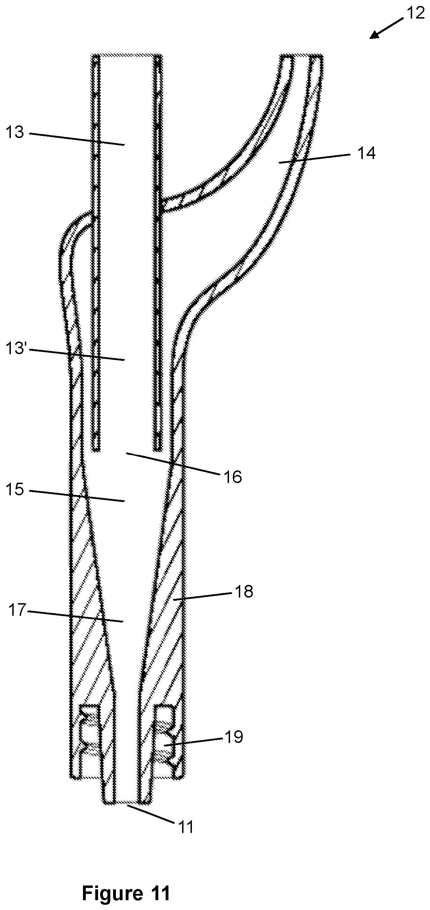

As shown in Figures 10 and 11, the flow converging chamber 15 and tapering section 17, the barrel 13/13', the second inlet 14, the outer wall 18 and the outlet 11 may be produced as a self-contained flow converger part 12, to which the first and second barrels 26, 36 and the needle 40 may subsequently be attached. Alternatively, the flow converger part 12 may be fabricated integrally with the first and second barrels 26, 36.

Optionally but advantageously the inlet barrel 13/13' may be made of metal, such as stainless steel, to enable it to be made as thin as possible (e.g. having a wall thickness of 0.5 mm) to reduce off-axis fluid velocities and mixing as the first and second fluids come together at the meeting point 16 within the flow converging chamber 15. The barrel 13/13' may then be inserted into the rest of the flow converger part 12, through a hole on the top of the flow converger part 12, and fixed (sealed) into place.

The other components of the flow converger part 12, and indeed the syringe 10 in general, may be made of inexpensive plastics materials using well-known mass manufacturing techniques such as injection moulding.

The function and configuration of the flow converger part 12 will now be described in greater detail with reference to Figures 11 to 17.

The objective of the flow converger part 12 is to produce a concentric flow stream of the first fluid 1 within the second fluid 2 at the outlet 11 (i.e. entrance to the needle 40). Ideally, the first and second fluids do not mix at all. In order to minimise the risk of the first and second fluids mixing, the flow converger part 12 provides the following characteristics:
- an axisymmetric pressure profile in the pathway of the second fluid 2, at the point where the streams of the first fluid 1 and the second fluid 2 meet (i.e. meeting point 16);
- a tapering section 17 leading to a minimum diameter section (outlet 11); and
- a predictable thickness of the second fluid (e.g. PBS) in the minimum diameter section (outlet 11).

The first two of these characteristics will now be described in more detail.

### - Axisymmetric pressure profile in the pathway of the second fluid 2, at the point where the streams of the first fluid 1 and the second fluid 2 meet (i.e. meeting point 16)

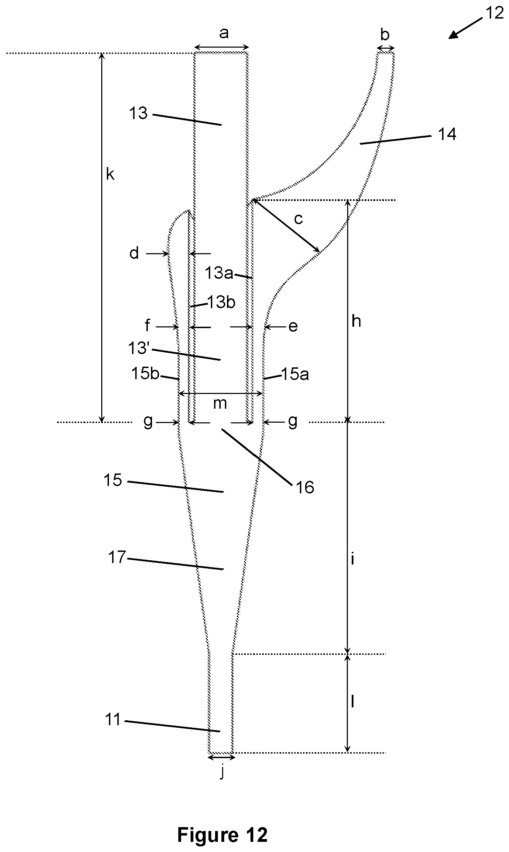

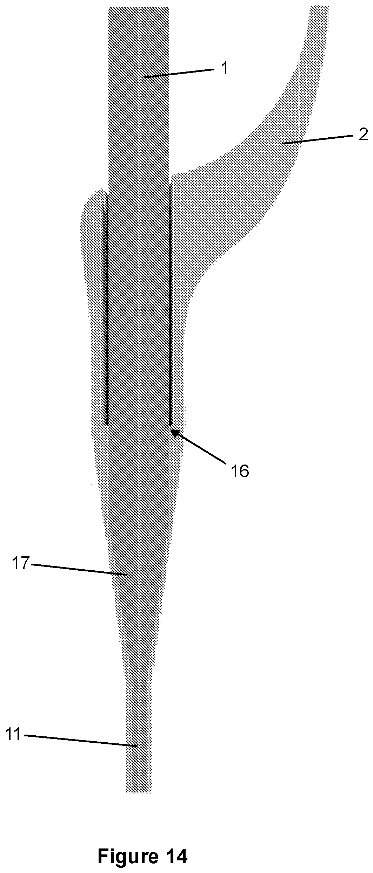

The "wrap-around" configuration of the flow converger 12 brings the second fluid 2 (e.g. PBS) into contact with the first fluid 1 (e.g. hydrogel) at the meeting point 16. With reference to Figure 14 (which corresponds to Figures 11 and 12 and illustrates the first and second fluids 1, 2 flowing through the flow converging chamber), if the second fluid 2 were to have an asymmetric pressure profile at the meeting point 16, it would create an imbalance in forces that would increase the likelihood of the two fluid streams mixing. Thus, the flow converger 12 is configured to provide an axisymmetric pressure profile in the second fluid 2 at the meeting point 16, such that there is minimal net driving force to cause mixing of the first and second fluids.

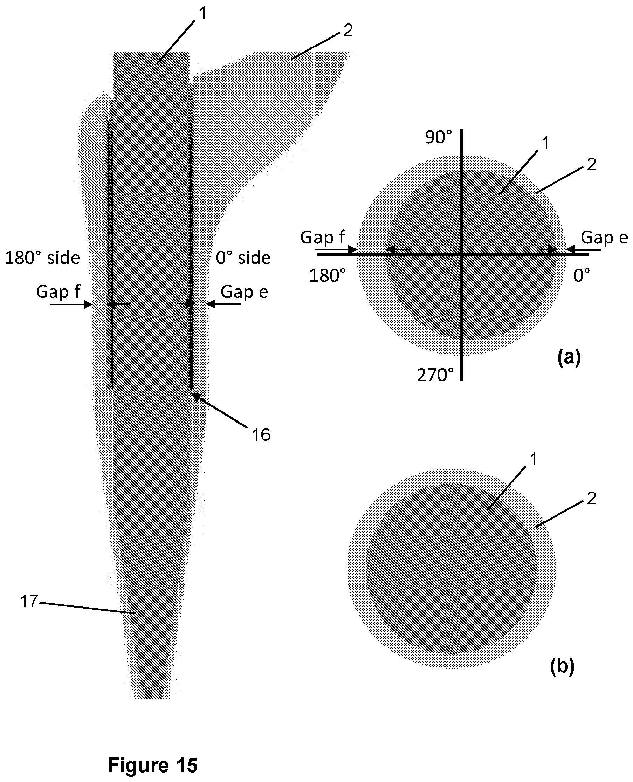

With reference to Figure 15, because the fluid on the "180° side" of the flow converger has travelled further than the fluid on the "0° side", if the narrow flow path of the flow converger (around part 13') were to have an axisymmetric shape then a larger pressure drop would likely be formed in the fluid pathway on the "180° side". In order to balance this, the gap f (flow path width) on the "180° side" is made slightly larger than the gap e (flow path width) on the "0° side", with the gap (flow path width) varying smoothly around the circumference. This is illustrated further in cross-section (a), in which the 0° and 180° positions correspond to said 0° side and said 180° side, and intermediate 90° and 270° positions are also shown. The pattern of gap (flow path) asymmetry may be designed using computational fluid dynamics modelling. Ideally, the gap (flow path width) is axisymmetric at the meeting point 16, as shown in cross-section (b). It may be noted that the gap (flow path width) at the meeting point 16 is referred to as gap g below.

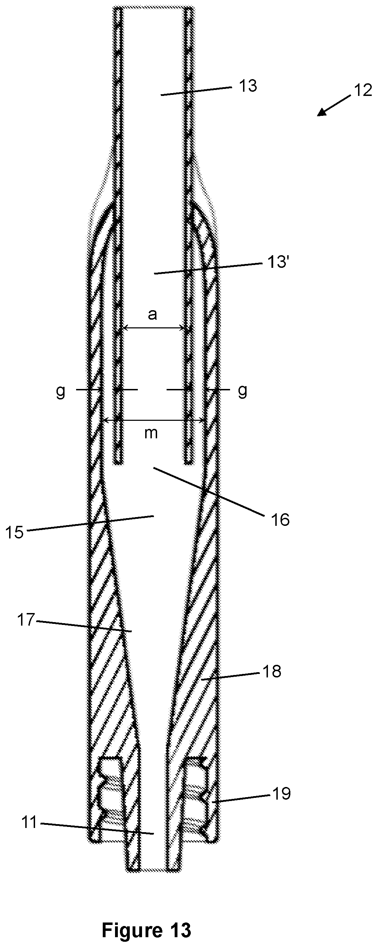

To illustrate the above principles in more detail, Figures 12 and 13 have been annotated to indicate exemplary non-limiting dimensions in respect of the flow converger part 12, which are as follows:
a = 4.80 mm
b = 1.50 mm
c = 8.85 mm
d = 1.83 mm
e = 1.02 mm
f = 1.09 mm
g = 0.95 mm
h = 20.51 mm
i = 21.30 mm
j = 2.10 mm
k = 34 mm
| = 9.40 mm
m = 7.70 mm

With reference in particular to Figure 12, in the illustrated configuration of the flow converger part 12 it will be appreciated that the second inlet 14 meets the flow converging chamber 15 on the first side of the flow converging chamber 15 (at the top of the flow converging chamber 15 upstream of the meeting point 16, and to one side of part 13'). The flow converging chamber 15 has a second side opposite the first side. The flow converging chamber 15 has a first flow path width e between a first side wall 13a of the first inlet (part 13') and an outer wall 15a of the flow converging chamber 15 on the first side. Further, the flow converging chamber 15 has a second flow path width f between a second side wall 13b of the first inlet (part 13') and an outer wall 15b of the flow converging chamber 15 on the second side. The second flow path width f is slightly greater than the first flow path width e. With the present exemplary dimensions, f = 1.09 mm whereas e = 1.02 mm, giving a difference of 0.07 mm.

The second flow path width increases from f in a direction away from the outlet 11, widening to a maximum width d at an upper part or shoulder of the flow converging chamber 15, opposite the point where the second inlet 14 meets the flow converging chamber 15 on the first side. With the present exemplary dimensions, d = 1.83 mm.

Thus, it will be appreciated that the upper part or shoulder of the outer wall 15b of the flow converging chamber 15 is skewed outwardly on the second side (the "180° side" referred to above), opposite the point where the second inlet 14 meets the flow converging chamber 15 on the first side (the "0° side" referred to above).

The second flow path width and the first flow path width decrease (from f and e respectively) towards the outlet 11. At the meeting point 16, the first flow path width and the second flow path width (both denoted by g in this position) become equal to each other. With the present exemplary dimensions, g = 0.95 mm.

This configuration (and without being limited to the above exemplary dimensions) enables an axisymmetric pressure profile to be achieved in the second fluid 2 at the meeting point 16.

### - Tapering section 17 leading to a minimum diameter section (outlet 11)

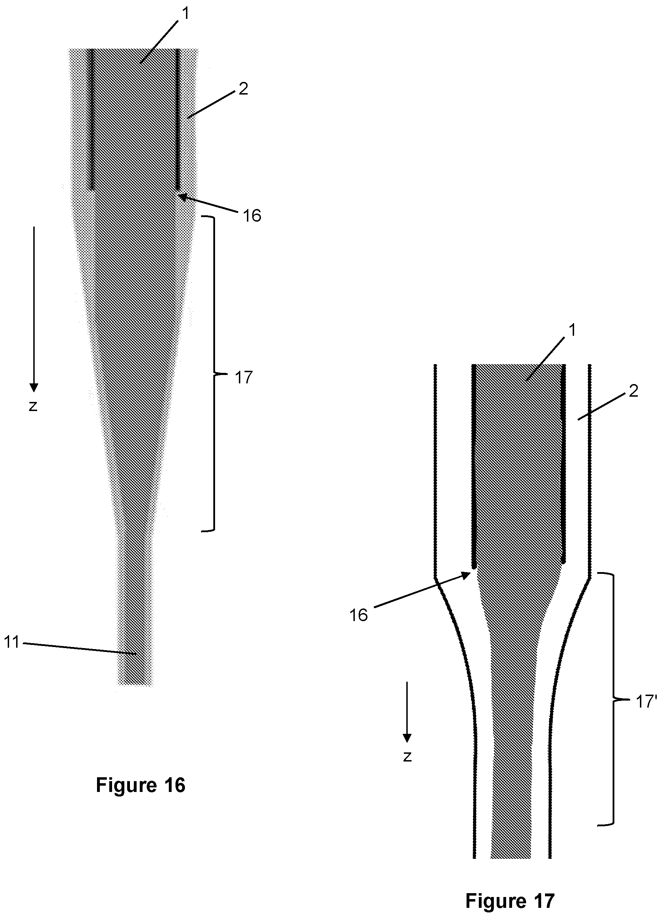

Most conventional syringe bodies feature sudden changes in diameter that would increase the likelihood of fluid mixing in the present application. However, with reference to Figure 16 (which shows the flowing first and second fluids 1, 2 within the tapering section 17), a gradual transition in diameter along the tapering section 17 minimises the risk of fluid mixing. In the case of Figure 16, the flow converging chamber tapers in a linear manner, which provides good results.

However, the tapering section 17 may be further modified to minimise the peak viscous normal stresses and strain rate encountered by the first fluid 1. This may have additional benefits of minimising the risk of fluid mixing as well as minimising the likelihood of cell damage. In this modified geometry, as sketched in Figure 17, the radius r of the tapering section is inversely proportional to the square root of the distance z along the tapering section towards the outlet 11, i.e. such that the radius r is substantially proportional to z^{-½}. This geometry has been derived from basic Newtonian fluid dynamic approximations; more complex computational flow modelling techniques could be used to refine this shape for particular fluids.

An additional means of minimising the shear rate encountered by the fluids flowing through the injection device 10 would be to render the inner surfaces "superhydrophobic" by some combination of surface charge, micropatterning or other techniques known to increase hydrophobicity. This would greatly decrease the wall shear rate, which is typically the highest value found in the cross-section of tubular flows of the type relevant to cell injection applications. The shear rate values throughout the rest of the cross-section would be reduced as well.

### Summary and further implementational details

We have discovered that cells are damaged during small-bore needle injections and the like as a result of excessive shear rate; not shear stress as has always been assumed [3, 4, 5]. In a laminar flow situation, shear rate expresses how fast one layer of fluid moves past another (picture sheets of plywood sliding relative to each other). Shear stress expresses the force required to move one layer of fluid over another. This important finding has provided a criterion on which to base the design of the present syringe 10, that can minimise cell deaths by avoiding excessive shear rates. Previous observations have indicated that a significant proportion of cells are damaged or killed during the injection process, which greatly reduces the effectiveness of cell-based therapies (including stem cell therapies). We project cell viability rates of 90% with our injector.

Thus, we have developed a two-chamber syringe 10 in which the inner fluid 1 (e.g. a hydrogel) exhibits a yield stress, and contains cells or other biological material(s) to be injected for medical, biological, research or manufacturing purposes. (Other examples of fluids that exhibit a yield stress include a Bingham plastic, Casson fluid, and toothpaste.) The outer fluid 2 (which may be a water-based solution, for example) provides a lubricating layer that reduces the shear rate to which the inner fluid 1 is subjected as it passes down the needle 40. Ideally, this results in the entirety of the inner fluid 1 being subjected to shear stresses lower than its yield stress. In this case, the inner fluid 1 would move as a solid core, and none of the cells in the inner fluid 1 would be lost to shear rate-induced damage. In this solid core, the shear rate is zero. The needle 40 may be of a size and shape typical of those currently used in medical, research and manufacturing processes.

As shown in Figures 9 and 10, arranging the first and second syringe barrels 26, 36 in a "side-by-side" configuration has the advantage of being able to produce a thinner layer of the second fluid 2 than if the syringe body itself possessed a concentric design (e.g. as in WO 2013/070692 A1). In the test cases we have explored, the required thickness of the second fluid 2 in the needle is less than 5% of the radius of the needle. In the present syringe 10, the first fluid plunger 23 is connected to, and parallel to, the second fluid plunger 33. The diameters of the two syringe barrels 26, 36 are sized to supply appropriate flow rates, with the plungers 23, 33 moving at the same velocity for ease of operation. The two fluids 1, 2, ejected from the first and second barrels 26, 36 respectively, are then combined in the converging chamber 15.

The upper wall of the converging chamber 15, where an incoming flow of the second fluid 2 is distributed around the circumference of the converging chamber 15 so as to surround the introduced flow of the first fluid 1 (via inlet barrel 13/13'), is designed such as to provide a layer of the second fluid 2 that has an axisymmetric pressure profile at the point 16 where the first and second fluids 1, 2 meet. This minimises the risk of the first and second fluids 1, 2 mixing, which could result in cells being transported from the inner fluid 1 to the outer fluid 2 where shear rates are high. Our presently-preferred way of achieving this, as illustrated, is to skew the upper wall of the flow converging chamber 15 outwardly, opposite the point where the second inlet 14 (conveying the second fluid 2) meets the flow converging chamber 15, such that the distribution of resistance to fluid flow around the circumference results in a uniform distribution of the second fluid 2, and subsequently gives the second fluid an axisymmetric pressure profile at the point 16 where it meets the first fluid 1.

However, in alternative embodiments, vanes or fins that swirl the second fluid 2 around the flow converging chamber 15 and then direct it toward the outlet 11 may be used instead, again with a view to creating pathways of identical (or near-identical) flow resistance.

The syringe 10 is designed to deliver the two fluids 1, 2 in a concentric manner into the needle 40 at a velocity and radial extents such that the yield stress of the inner fluid 2 is not exceeded at its outer surface. The syringe 10 is therefore designed with two adjacent barrels 26, 36 and ideally a combined plunger depressor surface (former by the interconnected plunger flanges 22, 32) for ease of manufacturing and deployment. Alternatively, though, it is possible to have plungers 23, 33 that move at different velocities, particularly if they are both driven by stepper motors.

The end of the syringe (tapering region 17) converges in such a way to minimise the risk of mechanical damage to cells in the inner fluid, and to maintain the fluids in a concentric geometry all the way to the entrance of the needle 40. Current syringes typically converge abruptly from a large diameter to a very small diameter, which would subject the fluids 1, 2 to high viscous normal stresses and might result in mixing of the two fluids. High viscous normal stress or normal strain rate may cause a similar risk of disrupting the cells within the inner fluid 1 to that of viscous shear strain rate. Mixing would reduce the advantage of the lubricating properties of the outer fluid 2 as the combined material moves through the needle 40.

As the plungers 23, 33 are pushed, the inner fluid 1, separated from the outer fluid 2 by a nominally cylindrically-shaped wall (barrel part 13'), will be subjected to a finite shear rate in order to start moving down the syringe. This is an unavoidable consequence of having the two fluids 1, 2 initially in separate compartments (barrels 26, 36) in the syringe. However, there is inherently less risk of subjecting the components of the inner fluid 1 to potentially damaging shear rates because the diameter of the first barrel 26 and the inlet barrel 13/13' is much larger than that of the needle 40. Only a small portion of the first fluid 1 very close to the wall of barrel 26 or of barrel 13/13' will be subjected to a potentially damaging shear rate. In this regard, it is possible to determine the shear rate at which cells are damaged using mechanobiological assays, such as injecting cells at known flow rates with water-based fluids and staining for markers of cell death and/or viability. Dextran can be added to increase viscosity so that shear rate and shear stress can be varied independently. We have done this, and the results (e.g. as presented in Figure 6) show that cell viability correlates with shear rate but not shear stress.

Finally, the tapering section 17 where the diameter of the flowing first fluid 1 transitions from substantially the diameter of the barrel 13/13' to the diameter of the needle 40 (specifically, to the internal diameter of the needle shaft 44) may be designed according to the following criteria:
A. Minimisation of the risk that the two fluid layers mix before entering the needle using (1) a shape that minimises the radial pressure gradient (long taper), or (2) a surface that separates the two fluids that is also shaped to provide a smooth transition from the syringe flow patterns to the needle flow patterns, or some combination of the two.
B. Maintenance of the viscous shear stress experienced by the first fluid 1 below a threshold value.
C. Minimisation of the viscous normal stress experienced by the first fluid 1, as described above.

### Modifications and alternatives

Detailed embodiments and some possible alternatives have been described above. As those skilled in the art will appreciate, a number of modifications and further alternatives can be made to the above embodiments whilst still benefiting from the inventions embodied therein.

For example, in the above-described embodiments the injection device is in the form of a multi-compartment syringe 10. However, it will be appreciated that the present principles (particularly with respect to the arrangement of the first and second barrels 26, 36 and the respective fluid paths) may be applied to types of injection devices other than syringes.

Moreover, in the above-described embodiments, the first fluid 1 (which contains the cells or other biological materials) is generally described as being different from the second (surrounding) fluid 2. For example, the first fluid 1 may be a hydrogel whereas the second fluid 2 may be PBS. However, in other embodiments, the first and second fluids may be the same, the only difference being that the first fluid contains the cells or other biological materials, whereas the second fluid does not. Whilst this does not enable the first and second fluids to have different properties in terms of viscosity and yield stress, etc., for some applications it may be sufficient simply to form an inner flow of a fluid containing biological materials, concentrically surrounded by an annular layer of the same fluid in which the biological materials are not present.

### References

[1] T. Rossetti, F. Nicholls and M. Modo, "Intracerebral Cell Implantation: Preparation and Characterization of Cell Suspensions", Cell Transplantation, vol. 25, no. 4, pp. 645-664, 2016.
[2] M.H. Amer, F.R. Rose, K.M. Shakesheff, M. Modo and L. J. White, "Translational considerations in injectable cell-based therapeutics for neurological applications: concepts, progress and challenges", npj Regenerative Medicine, vol. 2, no. 1, pp. 1-13, 2017.
[3] B.A. Aguado, W. Mulyasasmita, J. Su, K.J. Lampe and S.C. Heilshorn, "Improving viability of stem cells during syringe needle flow through the design of hydrogel cell carriers", Tissue Eng Part A 2012, 18:806-815.
[4] A. Blaeser, D.F. Duarte Campos, U. Puster, W. Richtering, M.M. Stevens and H. Fischer, "Controlling Shear Stress in 3D Bioprinting is a Key Factor to Balance Printing Resolution and Stem Cell Integrity", Adv Healthc Mater 2016, 5:326-333.
[5] M.H. Amer, F. Rose, K.M. Shakesheff and L.J. White, "A biomaterials approach to influence stem cell fate in injectable cell-based therapies", Stem Cell Res Ther, 2018. 9(1): p. 39.

## Claims

1. An injection device (10) comprising:
a first barrel (26) for containing a first fluid (1), and having a first plunger (23);
a second barrel (36) for containing a second fluid (2), and having a second plunger (33), the second barrel (33) being arranged to one side of the first barrel (26);
a flow converging chamber (15); a first inlet (13) and a second inlet (14); the flow converging chamber (15) being in fluid communication with the first and second barrels (26, 36), for receiving, in use, a flow of the first fluid (1) from the first barrel (26) via the first inlet (13) to the flow converging chamber (15) when the first plunger (23) is advanced within the first barrel (26), and a flow of the second fluid (2) from the second barrel (36) via the second inlet (14) to the flow converging chamber (15) when the second plunger (33) is advanced within the second barrel (36), simultaneously with the advancement of the first plunger (23), the first inlet (13) being arranged to introduce the flow of the first fluid (1) in a laminar concentric manner within the flow of the second fluid (2), at a meeting point (16) within the flow converging chamber (15), to produce a laminar concentric flow of the first fluid (1) within the second fluid (2); and
an outlet (11) of the flow converging chamber (15), for outwardly delivering the laminar concentric flow of the first fluid (1) within the second fluid (2);
wherein the first inlet (13) is in the form of a barrel, of which a downstream part (13') extends within the flow converging chamber (15), towards the outlet (11), and wherein the second inlet (14) is upstream of the meeting point (16) and alongside the downstream part (13') of the first inlet (13) and arranged such that, in use, at the meeting point (16), the first fluid (1) and the second fluid (2) are both already flowing in the direction of the outlet (11); and
**characterised in that**
the flow converging chamber (15) tapers towards the outlet (11), the taper of the flow converging chamber (15) beginning at, or downstream of, the meeting point (16).

2. The injection device according to claim 1, wherein the second barrel (36) is of smaller diameter than the first barrel (26).

3. The injection device according to claim 1 or claim 2, wherein the taper of the flow converging chamber (15) has a radius r that varies with distance z towards the outlet (11), in a manner such that the radius r is substantially proportional to z^{-½}.

4. The injection device according to any preceding claim, wherein the first inlet (13) and the outlet (11) are substantially axially aligned with the first barrel (26).

5. The injection device according to any preceding claim, wherein, when viewed in longitudinal cross section:
the second inlet (14) meets the flow converging chamber (15) on a first side of the flow converging chamber (15);
the flow converging chamber (15) has a second side opposite the first side;
the flow converging chamber (15) has a first flow path width (e) between a first side wall of the downstream part (13') of the first inlet (13) and an outer wall (15a) of the flow converging chamber (15) on the first side;
the flow converging chamber (15) has a second flow path width (f) between a second side wall of the downstream part (13') of the first inlet (13) and an outer wall (15b) of the flow converging chamber (15) on the second side; and
the second flow path width (f) is greater than the first flow path width (e);
optionally wherein the second flow path width (f-d) increases in a direction upstream from the meeting point (16), away from the outlet (11);
and optionally wherein the second flow path width (g) is substantially equal to the first flow path width (g) at the meeting point (16).

6. The injection device according to any preceding claim, wherein at least some of the internal surfaces are superhydrophobic.

7. The injection device according to any preceding claim, wherein the first and second barrels (26, 36) are parallel to one another.

8. The injection device according to any preceding claim, further comprising a needle (40) in fluid communication with the outlet (11).

9. The injection device according to any preceding claim, wherein the first and second plungers (23, 33) are connected to each another.

10. The injection device according to any preceding claim, wherein the first and second plungers (23, 33) are motorised.

11. The injection device according to any preceding claim, wherein the first fluid (1) contains biological materials such as biological cells.

12. The injection device according to any preceding claim, wherein the first fluid (1) has a higher viscosity than that of the second fluid (2); and/or
wherein the first fluid (1) exhibits a yield stress; and/or
wherein the first fluid (1) comprises a hydrogel; and/or
wherein the second fluid (2) comprises an aqueous fluid such as phosphate-buffered saline.

13. The injection device according to any preceding claim, wherein one or more of the first barrel (26), the first plunger (23), the second barrel (36), the second plunger (33) and the flow converging chamber (15) are formed of a plastics material.

14. The injection device according to any preceding claim, wherein the first inlet (13, 13') is formed of a metal such as stainless steel.

15. A non-therapeutic method of delivering a laminar concentric flow of a first fluid (1) within a second fluid (2), using an injection device (10) according to any preceding claim;
optionally wherein the first fluid (1) contains biological materials such as biological cells;
optionally wherein the first fluid (1) has a higher viscosity than that of the second fluid (2);
optionally wherein the first fluid (1) exhibits a yield stress;
optionally wherein the first fluid (1) comprises a hydrogel;
optionally wherein the second fluid (2) comprises an aqueous fluid such as phosphate-buffered saline;
preferably wherein, at the meeting point (16), the second fluid (2) has an axisymmetric pressure profile;
and preferably wherein the first fluid (1) is subjected to a shear rate of substantially zero as it passes through the flow converging chamber (15) and the outlet (11).

## Patentansprüche

1. Injektionsvorrichtung (10), die Folgendes umfasst:
einen ersten Zylinder (26) zum Enthalten eines ersten Fluids (1) und mit einem ersten Kolben (23);
einen zweiten Zylinder (36) zum Enthalten eines zweiten Fluids (2) und mit einem zweiten Kolben (33), wobei der zweite Zylinder (33) seitlich neben dem ersten Zylinder (26) angeordnet ist;
eine Flusszusammenlaufkammer (15); einen ersten Einlass (13) und einen zweiten Einlass (14); wobei die Flusszusammenlaufkammer (15) mit dem ersten und dem zweiten Zylinder (26, 36) in Fluidverbindung steht, um bei Verwendung einen Fluss des ersten Fluids (1) aus dem ersten Zylinder (26) über den ersten Einlass (13) zu der Flusszusammenlaufkammer (15), wenn der erste Kolben (23) innerhalb des ersten Zylinders (26) vorgeschoben wird, und einen Fluss des zweiten Fluids (2) aus dem zweiten Zylinder (36) über den zweiten Einlass (14) zu der Flusszusammenlaufkammer (15) aufzunehmen, wenn der zweite Kolben (33) innerhalb des zweiten Zylinders (36) gleichzeitig mit dem Vorschieben des ersten Kolbens (23) vorgeschoben wird, wobei der erste Einlass (13) angeordnet ist, um den Fluss des ersten Fluids (1) in einer laminaren konzentrischen Weise innerhalb des Flusses des zweiten Fluids (2) an einem Treffpunkt (16) innerhalb der Flusszusammenlaufkammer (15) einzuführen, um einen laminaren konzentrischen Fluss des ersten Fluids (1) innerhalb des zweiten Fluids (2) zu produzieren; und
einen Auslass (11) der Flusszusammenlaufkammer (15) zum Abgeben des laminaren konzentrischen Flusses des ersten Fluids (1) innerhalb des zweiten Fluids (2) nach außen;
wobei der erste Einlass (13) die Form eines Zylinders aufweist, von dem sich ein stromabwärts gelegener Teil (13') in die Flusszusammenlaufkammer (15) in Richtung des Auslasses (11) erstreckt, und wobei der zweite Einlass (14) stromaufwärts des Treffpunkts (16) und neben dem stromabwärts gelegenen Teil (13') des ersten Einlasses (13) angeordnet ist und derart angeordnet ist, dass bei Verwendung an dem Treffpunkt (16) sowohl das erste Fluid (1) als auch das zweite Fluid (2) bereits in die Richtung des Auslasses (11) fließen; und
**dadurch gekennzeichnet, dass** sich die Flusszusammenlaufkammer (15) in Richtung des Auslasses (11) verjüngt, wobei die Verjüngung der Flusszusammenlaufkammer (15) an dem oder stromabwärts des Treffpunkts (16) beginnt.

2. Injektionsvorrichtung nach Anspruch 1, wobei der zweite Zylinder (36) einen kleineren Durchmesser aufweist als der erste Zylinder (26).

3. Injektionsvorrichtung nach Anspruch 1 oder 2, wobei die Verjüngung der Flusszusammenlaufkammer (15) einen Radius r aufweist, der sich von dem Abstand z in Richtung des Auslasses (11) auf eine Weise unterscheidet, sodass der Radius r im Wesentlichen proportional zu z^{-½} ist.

4. Injektionsvorrichtung nach einem der vorhergehenden Ansprüche, wobei der erste Einlass (13) und der Auslass (11) im Wesentlichen axial an dem ersten Zylinder (26) ausgerichtet sind.

5. Injektionsvorrichtung nach einem der vorhergehenden Ansprüche, wobei, wenn im Längsquerschnitt betrachtet:
der zweite Einlass (14) an einer ersten Seite der Flusszusammenlaufkammer (15) auf die Flusszusammenlaufkammer (15) trifft;
die Flusszusammenlaufkammer (15) eine der ersten Seite gegenüberliegende zweite Seite aufweist;
die Flusszusammenlaufkammer (15) auf der ersten Seite eine erste Flusswegbreite (e) zwischen einer ersten Seitenwand des stromabwärts gelegenen Teils (13') des ersten Einlasses (13) und einer Außenwand (15a) der Flusszusammenlaufkammer (15) aufweist;
die Flusszusammenlaufkammer (15) auf der zweiten Seite eine zweite Flusswegbreite (f) zwischen einer zweiten Seitenwand des stromabwärts gelegenen Teils (13') des ersten Einlasses (13) und einer Außenwand (15b) der Flusszusammenlaufkammer (15) aufweist; und
die zweite Flusswegbreite (f) größer ist als die erste Flusswegbreite (e);
wobei die zweite Flusswegbreite (f-d) optional in einer Richtung stromaufwärts von dem Treffpunkt (16) weg von dem Auslass (11) zunimmt;
und wobei optional die zweite Flusswegbreite (g) an dem Treffpunkt (16) im Wesentlichen der ersten Flusswegbreite (g) entspricht.

6. Injektionsvorrichtung nach einem der vorhergehenden Ansprüche, wobei wenigstens einige der internen Oberflächen superhydrophob sind.

7. Injektionsvorrichtung nach einem der vorhergehenden Ansprüche, wobei der erste und der zweite Zylinder (26, 36) parallel zueinander sind.

8. Injektionsvorrichtung nach einem der vorhergehenden Ansprüche, die ferner eine Nadel (40) umfasst, die in Fluidverbindung mit dem Auslass (11) steht.

9. Injektionsvorrichtung nach einem der vorhergehenden Ansprüche, wobei der erste und der zweite Kolben (23, 33) miteinander verbunden sind.

10. Injektionsvorrichtung nach einem der vorhergehenden Ansprüche, wobei der erste und der zweite Kolben (23, 33) motorisch angetrieben sind.

11. Injektionsvorrichtung nach einem der vorhergehenden Ansprüche, wobei das erste Fluid (1) biologisches Material, wie biologische Zellen, enthält.

12. Injektionsvorrichtung nach einem der vorhergehenden Ansprüche, wobei das erste Fluid (1) eine höhere Viskosität aufweist als die des zweiten Fluids (2); und/oder
wobei das erste Fluid (1) eine Fließspannung aufweist; und/oder
wobei das erste Fluid (1) ein Hydrogel umfasst; und/oder
wobei das zweite Fluid (2) ein wässriges Fluid wie eine phosphatgepufferte Kochsalzlösung umfasst.

13. Injektionsvorrichtung nach einem der vorhergehenden Ansprüche, wobei eines oder mehrere des ersten Zylinders (26), des ersten Kolbens (23), des zweiten Zylinders (36), des zweiten Kolbens (33) und der Flusszusammenlaufkammer (15) aus einem Kunststoffmaterial ausgebildet ist/sind.

14. Injektionsvorrichtung nach einem der vorhergehenden Ansprüche, wobei der erste Einlass (13, 13') aus einem Metall wie Edelstahl ausgebildet ist.

15. Nicht therapeutisches Verfahren zum Abgeben eines laminaren konzentrischen Flusses eines ersten Fluids (1) innerhalb eines zweiten Fluids (2) unter Verwendung einer Injektionsvorrichtung (10) nach einem der vorhergehenden Ansprüche;
wobei das erste Fluid (1) optional biologische Materialien wie biologische Zellen enthält;
wobei das erste Fluid (1) optional eine höhere Viskosität aufweist als die des zweiten Fluids (2);
optional wobei das erste Fluid (1) eine Fließspannung aufweist;
optional wobei das erste Fluid (1) ein Hydrogel umfasst;
optional wobei das zweite Fluid (2) ein wässriges Fluid wie eine phosphatgepufferte Kochsalzlösung umfasst;
vorzugsweise wobei das zweite Fluid (2) an dem Treffpunkt (16) ein achsensymmetrisches Druckprofil aufweist;
und vorzugsweise, wobei das erste Fluid (1), wenn es durch die Flusszusammenlaufkammer (15) und den Auslass (11) passiert, einer Schergeschwindigkeit von im Wesentlichen Null ausgesetzt ist.

## Revendications

1. Dispositif d'injection (10) comprenant :
un premier cylindre (26) destiné à contenir un premier fluide (1) et ayant un premier piston (23) ;
un deuxième cylindre (36) destiné à contenir un deuxième fluide (2), et ayant un deuxième piston (33), le deuxième cylindre (33) étant disposé sur un côté du premier cylindre (26) ;
une chambre de convergence d'écoulement (15) ; une première entrée (13) et une deuxième entrée (14) ; la chambre de convergence d'écoulement (15) étant en communication fluidique avec les premier et deuxième cylindres (26, 36), pour recevoir, lors de l'utilisation, un écoulement du premier fluide (1) en provenance du premier cylindre (26) par l'intermédiaire de la première entrée (13) vers la chambre de convergence d'écoulement (15) lorsque le premier piston (23) est avancé à l'intérieur du premier cylindre (26), et un écoulement du deuxième fluide (2) en provenance du deuxième cylindre (36) par l'intermédiaire de la deuxième entrée (14) vers la chambre de convergence d'écoulement (15) lorsque le deuxième piston (33) est avancé à l'intérieur du deuxième cylindre (36), simultanément à l'avancement du premier piston (23), la première entrée (13) étant agencée pour introduire l'écoulement du premier fluide (1) de manière concentrique laminaire au sein de l'écoulement du deuxième fluide (2), au niveau d'un point de rencontre (16) à l'intérieur de la chambre de convergence d'écoulement (15), afin de produire un écoulement concentrique laminaire du premier fluide (1) au sein du deuxième fluide (2) ; et
une sortie (11) de la chambre de convergence d'écoulement (15), pour acheminer vers l'extérieur l'écoulement concentrique laminaire du premier fluide (1) au sein du deuxième fluide (2) ;
dans lequel la première entrée (13) est sous la forme d'un cylindre, dont une partie en aval (13') s'étend dans la chambre de convergence d'écoulement (15), en direction de la sortie (11), et dans lequel la deuxième entrée (14) est en amont du point de rencontre (16) et le long de la partie en aval (13') de la première entrée (13) et agencée de sorte que, lors de l'utilisation, au niveau du point de rencontre (16), le premier fluide (1) et le deuxième fluide (2) s'écoulent tous deux dans la direction de la sortie (11) ; et
**caractérisé en ce que** la chambre de convergence d'écoulement (15) s'amincit en direction de la sortie (11), le rétrécissement de la chambre de convergence d'écoulement (15) commençant au point de rencontre (16) ou en aval de celui-ci.

2. Dispositif d'injection selon la revendication 1, dans lequel le deuxième cylindre (36) est de plus petit diamètre que le premier cylindre (26).

3. Dispositif d'injection selon la revendication 1 ou la revendication 2, dans lequel le rétrécissement de la chambre de convergence d'écoulement (15) a un rayon r qui varie avec la distance z en direction de la sortie (11), de sorte que le rayon r est sensiblement proportionnel à z^{-½}.

4. Dispositif d'injection selon l'une quelconque des revendications précédentes, dans lequel la première entrée (13) et la sortie (11) sont sensiblement alignées axialement avec le premier cylindre (26).

5. Dispositif d'injection selon l'une quelconque des revendications précédentes, dans lequel, lors de l'observation en coupe longitudinale :
la deuxième entrée (14) rejoint la chambre de convergence d'écoulement (15) au niveau d'un premier côté de la chambre de convergence d'écoulement (15) ;
la chambre de convergence d'écoulement (15) a un deuxième côté opposé au premier côté ;
la chambre de convergence d'écoulement (15) a une première largeur de trajet d'écoulement (e) entre une première paroi latérale de la partie en aval (13') de la première entrée (13) et une paroi externe (15a) de la chambre de convergence d'écoulement (15) sur le premier côté ;
la chambre de convergence d'écoulement (15) a une deuxième largeur de trajet d'écoulement (f) entre une deuxième paroi latérale de la partie en aval (13') de la première entrée (13) et une paroi externe (15b) de la chambre de convergence d'écoulement (15) sur le deuxième côté ; et
la deuxième largeur de trajet d'écoulement (f) est supérieure à la première largeur de trajet d'écoulement (e) ;
éventuellement dans lequel la deuxième largeur de trajet d'écoulement (f-d) augmente selon une direction en amont du point de rencontre (16), s'éloignant de la sortie (11) ;
et éventuellement dans lequel la deuxième largeur de trajet d'écoulement (g) est sensiblement égale à la première largeur de trajet d'écoulement (g) au niveau du point de rencontre (16).

6. Dispositif d'injection selon l'une quelconque des revendications précédentes, dans lequel au moins certaines des surfaces internes sont superhydrophobes.

7. Dispositif d'injection selon l'une quelconque des revendications précédentes, dans lequel les premier et deuxième cylindres (26, 36) sont parallèles l'un à l'autre.

8. Dispositif d'injection selon l'une quelconque des revendications précédentes, comprenant en outre une aiguille (40) en communication fluidique avec la sortie (11).

9. Dispositif d'injection selon l'une quelconque des revendications précédentes, dans lequel les premier et deuxième pistons (23, 33) sont reliés l'un à l'autre.

10. Dispositif d'injection selon l'une quelconque des revendications précédentes, dans lequel les premier et deuxième pistons (23, 33) sont motorisés.

11. Dispositif d'injection selon l'une quelconque des revendications précédentes, dans lequel le premier fluide (1) contient des matériels biologiques tels que des cellules biologiques.

12. Dispositif d'injection selon l'une quelconque des revendications précédentes, dans lequel le premier fluide (1) a une viscosité supérieure à celle du deuxième fluide (2) ; et/ou
dans lequel le premier fluide (1) présente une contrainte d'élasticité ; et/ou
dans lequel le premier fluide (1) comprend un hydrogel ; et/ou
dans lequel le deuxième fluide (2) comprend un fluide aqueux tel qu'une solution saline tamponnée au phosphate.

13. Dispositif d'injection selon l'une quelconque des revendications précédentes, dans lequel un ou plusieurs éléments parmi le premier cylindre (26), le premier piston (23), le deuxième cylindre (36), le deuxième piston (33) et la chambre de convergence d'écoulement (15) sont formés d'une matière plastique.

14. Dispositif d'injection selon l'une quelconque des revendications précédentes, dans lequel la première entrée (13, 13') est formée d'un métal tel que l'acier inoxydable.

15. Procédé non thérapeutique de fourniture d'un écoulement concentrique laminaire d'un premier fluide (1) au sein d'un deuxième fluide (2), à l'aide d'un dispositif d'injection (10) selon l'une quelconque des revendications précédentes ;
éventuellement dans lequel le premier fluide (1) contient des matériels biologiques tels que des cellules biologiques ;
éventuellement dans lequel le premier fluide (1) a une viscosité supérieure à celle du deuxième fluide (2) ;
éventuellement dans lequel le premier fluide (1) présente une contrainte d'élasticité ;
éventuellement dans lequel le premier fluide (1) comprend un hydrogel ;
éventuellement dans lequel le deuxième fluide (2) comprend un fluide aqueux tel qu'une solution saline tamponnée au phosphate ;
préférentiellement dans lequel, au niveau du point de rencontre (16), le deuxième fluide (2) a un profil de pression axisymétrique ;
et préférentiellement dans lequel le premier fluide (1) est soumis à un taux de cisaillement sensiblement nul lorsqu'il passe à travers la chambre de convergence d'écoulement (15) et la sortie (11).
